# EUROPEAN PATENT SPECIFICATION

(11) **EP 0 154 664 B1**
(45) Date of publication and mention of the grant of the patent: **20.01.1993**
(21) Application number: 84103308.7
(22) Date of filing: 04.08.1980
(51) Int. Cl.: A61B 5/00, C12Q 1/26

(54) **Measurement of body substances**
Messung von Körpersubstanzen
Mesure de substances corporelles

(30) Priority: 02.08.1979 US 63159
(43) Date of publication of application: 18.09.1985
(62) Divisional of application: 80302661.6
(73) Proprietor: THE CHILDREN'S HOSPITAL MEDICAL CENTER, Cincinnati Ohio 45229 (US)
(72) Inventor: Clark, Leland C., Jr., Cincinnati Ohio 45220 (US)
(74) Representative: Bowman, Paul Alan

(56) References cited:
- FR-A- 2 338 495
- US-A- 3 542 662
- US-A- 4 005 700
- US-A- 4 020 830
- US-A- 4 071 020

## Description

This invention relates to a cutaneous method of analysing body substrates.

Instruments capable of continuously indicating the chemical composition of blood have proved to be useful in regulating operative and post-operative managements of patients, and in teaching and research. At first, such instruments were used with sensors mounted directly in the extracorporeal blood circuit that is used for perfusion of open-heart surgery patients. Later, continuous monitoring of both machine and patients was conducted by means of continuous withdrawal of blood pumped into external cuvettes equipped with appropriate sensors. Satisfactory systems are now provided for a rapid and accurate measurement of blood composition such as pH, CO₂ and pO₂.

In addition to the analytical techniques mentioned above, oxygen and carbon dioxide have been measured on the skin by virtue of their diffusing through it. Recently, the continuous monitoring of blood oxygen by a heated electrode positioned on hyperemic skin has been accomplished. Substances such as halogenated organic compounds, particularly fluorinated compounds, have also been found to diffuse through the skin and have been measured. For example, United States Patent Specification No. 3 911 138 discloses a method of taking quantitative measurements of skin-diffused fluorinated compounds by gas chromatography and electron-capture dectectors.

Other techniques have been employed for measuring biological substances in the blood. For example, ethanol is currently measured in blood, either directly or by breath sampling or, by classical chemical, gas chromatographic and enzyme methods. One of the alcohol enzyme methods depends upon the polarographic measurement of hydrogen peroxide, while others depend upon the consumption of oxygen. For example, British Patent No. 1507810 discloses a method for measuring the level of ethanol in a body fluid, such as blood, comprising providing a predetermined volume of body fluid, oxidising ethanol in the body fluid using an alcohol oxidase in a suitable buffer in vitro in the presence of excess molecular oxygen and measuring the rate of oxygen consumption, the oxidation taking place in the presence of an agent, e.g., a peroxide enzyme, able to suppress the formation of oxygen by peroxide decomposition. The rate of oxygen consumption is typically measured using an oxygen electrode. However, none of these methods readily lend themselves to continuous monitoring.

U.S. Patent Specification No. 4,071,020 discloses a method and apparatus for the in vivo measurement of substances on the epidermis or mucous membranes of a patient by the application of a suitable reagent to the skin surface followed by detection of the reaction products. The only enzymes specifically exemplified are glucose oxidase, glucose-6-phosphate deydrogenase, 6-phosphogluconic acid dehydrogenase, isocitric acid dehydrogenase and lactic acid dehydrogenase.

U.S. Patent Specification No. 3,542,662 discloses an enzyme electrode which must be placed in direct contact with the body fluid or tissue for the enzyme substrate in the fluid or tissue to be measured by the detection of oxygen. The electrode comprises an electromechanical transducer, suitable for in vivo biomedical applications, constructed by polymerisizing a gelatinous membrane of an enzyme gel over the measuring surface of an oxygen-sensing device, such as a polarographic oxygen electrode. The only enzyme specifically exemplified is glucose oxidase.

U.S. Patent Specification No. 4,020,830 also discloses an enzyme electrode which must be contacted with a solution containing the enzyme substrate. The device can be in the form of a needle for insertion into a tissue, when the method performed is invasive. The only enzymes specifically exemplified are glucose oxidase, lactate oxidase and β-glucosidase.

In summary, while there are a variety of techniques available for the measurement of blood gases and other substances, new methods are desired which more readily lend themselves to continuous monitoring or enable the non-invasive measurement of key non-gaseous biological substances.

European Patent No. 24155 discloses apparatus suitable for use in a cutaneous method of analysing body substrates comprising:
1. an enzyme implant which is adapted for insertion into the body of a mammal and contains an enzyme which is reactable with a non-gaseous substrate in the body, which substrate is to be measured, to produce a detectable change in the skin of the mammal, and
2. analysing means for analysing said change in the skin, without invading the body at the time of detection, as a measure of the amount of said non-gaseous substrate in the body.

According to the present invention there is provided a method of detecting for the presence in the body of a non-gaseous substrate which is volatilizable through the skin, which method comprises introducing at the surface of the skin an enzyme selected for the substrate to be detected for a sufficient time for any of the substrate present to react with the enzyme and detecting a condition of the skin as an indication of the presence of the substrate in the body.

The present invention provides a method for cutaneously measuring non-gaseous substances present in the body and which are volatilizable through the skin. The method is conducted by contacting the substrate through the skin, for example, of a mammal, with an enzyme selective for that substrate, reacting the substrate with the enzyme and directly detecting a condition of the skin as a measure of the amount of substrate. The procedure may be completely non-invasive.

In a most preferred method, the skin is arterialized and the enzyme is reacted with the substrate at the skin surface. A condition of the reaction is detected such as the amount of oxygen consumed, or hydrogen peroxide or carbon dioxide by-products as a measure of the amount of substance. For example, the amount of oxygen may be measured by a skin-contact oxygen electrode, particularly a heated electrode. This electrode is sometimes referred to herein simply as a transcutaneous oxygen electrode or tcp0₂ electrode. The heat arterializes the capilliaries in the skin, that is to say, the blood in the skin is brought into equilibrium with the blood in the arteries. Quantitative measurements may then be made. The skin capilliaries may also be arterialized by chemical treatment.

In an alternative method, the enzyme may react with a substance to produce by-product hydrogen peroxide which may then be sensed by a hydrogen peroxide sensitive electrode. For example, an H₂O₂ polarographic anode may be employed to detect subdermal components. Thus, a transcutaneous tcpO₂, tcpH₂O₂, or even a tcpCO₂ electrode may be employed as the skin condition analyzer.

In addition to positioning polarographic electrodes on hyperemic skin to detect oxygen in a local subdermal oxygen sink or by-product hydrogen peroxide, other procedures for quantitation of the substrate may be employed. For example, a colorimetric method may be used for detecting amounts of hydrogen peroxide produced by enzymatic reaction. The amount of hydrogen peroxide produced may be measured by a system which comprises a chromogenic reagent or reagents capable of undergoing a colour change in the presence of hydrogen peroxide, the amount of hydrogen peroxide present being measured by colorimetrically measuring the colour change. One known method of such measurement is by means of a quadravalent-titanium and xylenol orange which react to form a stable red colour with hydrogen peroxide (Taurenes & Nordschow, Amer. J. Clin. Path., 1968, 49, 613). Reference may be made to this article for details and to United States Patent Specification No. 3 907 645 for suitable reactants. The amount of hydrogen peroxide produced is measured by the intensity of the colour.

Furthermore, the enzyme may be tattooed in the skin. In this form an enzyme or a detector of the enzyme reaction may be immobilized in the skin and a colour change or a condition of the skin may be visually observed or measured.

The reaction of the enzyme with the substance being measured may also be followed through the skin by measuring the electrons which are removed during the enzymatic reaction and transferred, for example, to a coloured dye.

In its broadest aspect, the present invention is directed to a method for the cutaneous measurement of a non-gaseous, volatilizable substance by reacting an enzyme with the substance anywhere across the layer of the skin and detecting a condition of the skin as a measure of the amount of substance. The enzyme is placed on the skin in accordance with any particular technique. One particularly preferred technique is disclosed in European Patent No. 24155 in which the enzyme is implanted below the skin. The implantation allows for continuous monitoring of the substance under examination. Although, the exact nature of healing, fibrous tissue invasion and capilliary new growth following implantation cannot, of course, be controlled, the implantation can be regulated with satisfactory practical limits. The tissue reaction to such implants in humans after many months is small and they are easily replaced and removed. Sensing may be accomplished by relating the difference in the polarographic oxygen current between the normal skin and the enzyme modulated skin. For example, reference may be made to prior U.S. Patent Specification Nos. 3,912,386, 3,380,905 and 3,539,455 for specific electrode structures which may be used to detect oxygen and H₂0₂. Using devices of this type, a dual electrode system may be used by relating the difference in polarographic oxygen current between the normal skin and the enzyme modulated skin. In another form a single electrode system can be employed. For example, polarographic anodes of the type disclosed in U.S. Patent Specification No. 4,040,908 may be employed to measure hydrogen peroxide by-product as a measure of the substrate.

According to one procedure, the enzyme may be dissolved in water and injected just under, or into, the skin and a tcp0₂ electrode secured on the skin just over the enzyme site. The temperature of the skin is preferably controlled at approximately 38 to 44°C. In another procedure, the enzyme may be mixed with silicone and fluorocarbon oils before subcutaneous injection. Enzyme has also been mixed with silicone monomer and converted by a suitable catalyst to a thin rubber-like polymer sheet about half the size of a postage stamp which is then implanted through an incision in the skin. Such implants heal rapidly and retain enzyme activity for many days or weeks and probably much longer. Enzyme implants have been made using a thin sheet of reinforced SILASTIC (organosilicone polymers, Dow Corning subdermal implant No. 501-1.007 inches thick) coated with enzyme, immobilized by treatment with glutaraldehyde solution and drying in the cold. Enzyme, either free or immobilized has also been trapped between two layers of cellophane, cuprophan or collagen prior to implantation.

Therefore, various modes of cutaneous treatment, including implantation, and devices for achieving same may be employed in the above described methods. Skin implants may be very small, e.g. a sphere with a minimum dimension of about 1mm in diameter. Patients who require continuous monitoring may be provided with a skin implant and their condition may be continuously monitored by any of the aforementioned detection techniques. Furthermore, in another form, the encapsulated enzyme is embodied below the surface of the skin in such a way that it is visible.

The invention will now be described with reference to the accompanying drawings and the following Example, in which:
Figure 1 illustrates a typical transcutaneous electrode arrangement for detecting skin oxygen contact as a measure of the substrate, and
Figure 2 is a plot showing measurement of ethanol with a transcutaneous p0₂ electrode.

### Example

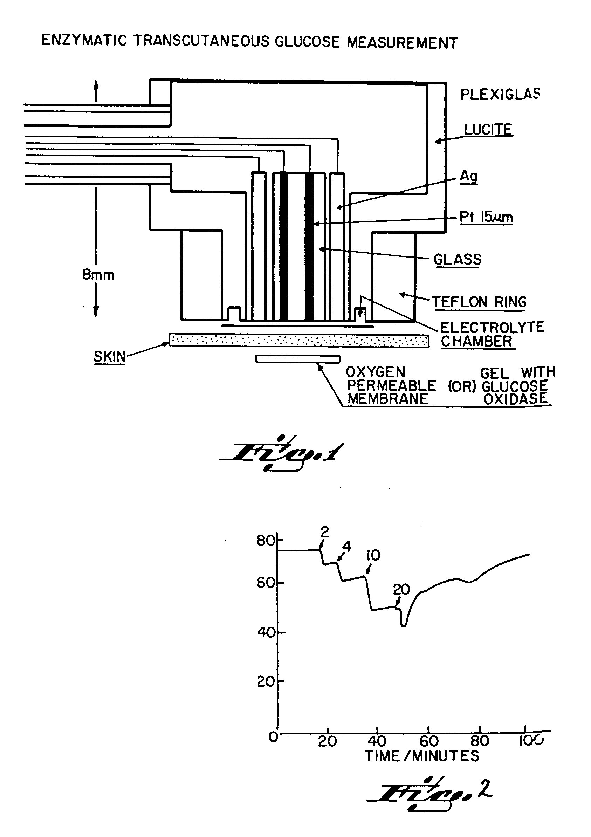

In this series of experiments, the oxygen-consuming alcohol oxidase was placed on the skin of an anesthetized cat. The animal was anesthetized with sodium pentobarbital and maintained at 38^{o}C with an infrared heater modulator modulated by a rectal thermistor signal. The electrode was fastened to the shaved skin just below the thorax. A few crystals of the oxidase preparation in about 50 µl of water (215 mg/50 µl) was placed on the skin and the electrode was set in place. After a stable reading was obtained, alcohol solution was injected. The results are shown in Figure 2 and numbers referred to at the points of injection, namely 2, 4, 10 and 20, are the number of millilitres of 10% ethanol given intraveneously. The measurements of the circulating ethanol were employed using a transcutaneous tcpO₂ electrode of the type shown in Figure 1. With reference to Figure 2, it is demonstrated that increasing amounts of alcohol decreased the tcpO₂ step-wise and that recovery toward the initial value occurred over the following minutes. The less than expected effect of the 20 millilitre dose was not understood, but may possibly be due to a pharmacological affect on the skin or possibly an acute drop in blood pressure.

Other means of performing the experiments of the above type involving volatilizable components, such as alcohol, include the incorporation of the enzyme in the electrode's electrolyte, immobilizing it on the membrane, and the use of two cathodes, one coated with enzyme and one uncoated. As mentioned above, one may also have a coated and an enzyme-free spot on the skin and calibrate by measuring the pO₂ versus blood or end tidal alcohol. The temperature control required for the tcpO₂ measurement is ideal for stabilizing enzyme activity. Enzyme would be best dissolved in a buffer with suitable coenzymes and stabilizing agents. There are several alcohol oxidases and dehydrogenases with varying specificity toward alcohols of different chemical structures, but all respond to ethanol for use in the Experiments. The tcpO₂ skin procedure above discussed with reference to alcohol can be used for the continuous measurement of other volatile enzyme substrates where oxygen depletion is utilized in their measurement.

In view of the above experiments, it is obvious that a number of other enzymes can be used in order to detect and measure a volatilizable substance transcutaneously. The following Table is a listing of the enzymes, their identifying number, source and typical substrates with which they may react for measurement in accordance with the invention.

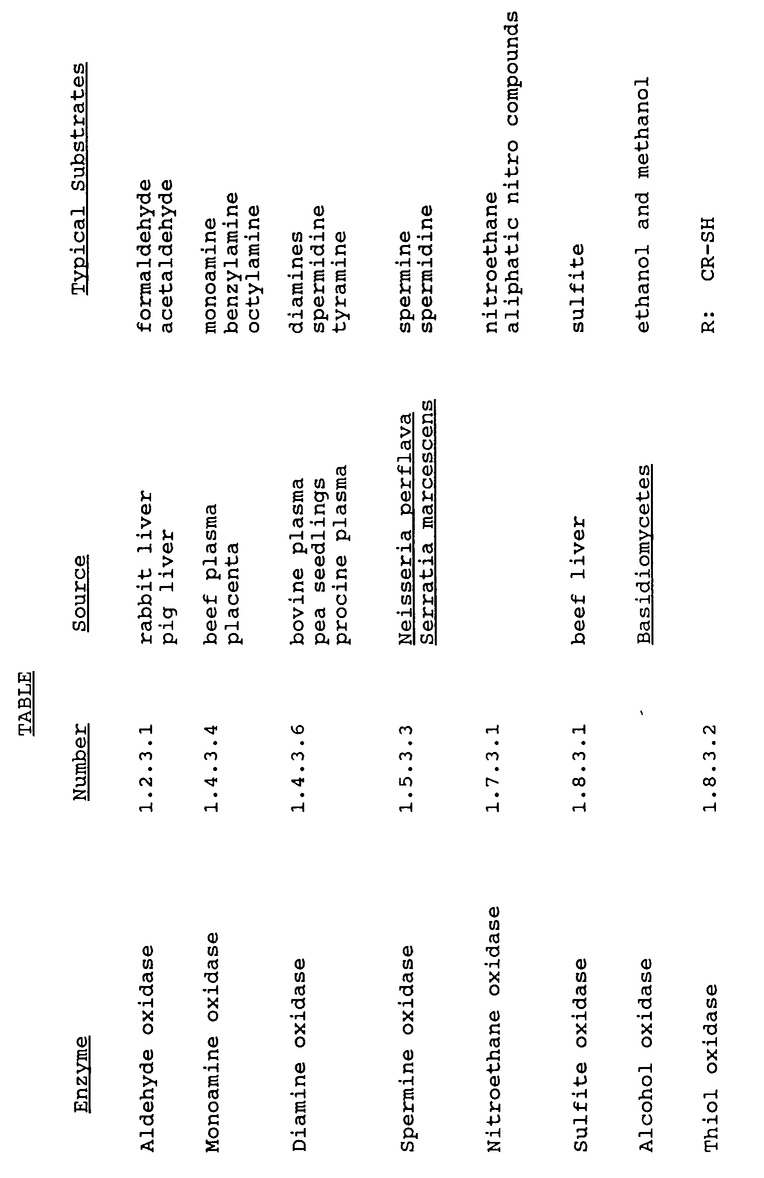

Any enzyme may be used which, in the process of catalyzing the reaction with its substrate or substrates directly or indirectly, consumes or requires oxygen.

Using the international nomenclature of the enzyme commission (see for example T.E. Barman, Enzyme Handbook, Vol. 1, 2, and Supplement, Springer-Verlag, New York, 1969), classes of enzymes can be described which are useful in the above described method. Since new enzymes are regularly discovered, examples of present known enzymes can be used to illustrate the principles involved. There are six main classes:
1. Oxidoreductases
2. Transferases
3. Hydrolases
4. Lysases
5. Isomerases
6. Ligases.

Most of the oxygen consuming enzymes are in Class 1. If such enzymes may use molecular oxygen directly, they are then called oxygen oxidoreductases, or if indirectly, through a "coenzyme" or "cofactor" which is reduced by the enzyme and reoxidized by molecular oxygen, they are simply called oxidoreductases.

Class 1, the oxidoreductases, are divided into sub-classes, for example, 1.1 are those acting on the CH-OH group of donors. Class 1.1 is divided as follows:
1.1.1. with NAD or NADP as acceptor
1.1.1. with cytochrome as acceptor
1.1.3. with oxygen as acceptor
1.1.99 with other acceptors

The other five main classes of enzymes can be used in conjunction with the oxidoreductases, or oxygen-consuming dehydrogenases, to expand the range of analysis.

In performing the techniques described above, it should be understood that foreign or other proteins injected subcutaneously are absorbed rapidly. Proteolytic enzymes may destroy the enzyme or it may be picked up by Kupffer cells. If the immune system of the body including the opsonins can contact the enzyme, it will be marked for destruction. Some antibodies attach to enzymes (Freund's adjuvant is used to mix with the enzyme before injection) and they are inactivated by antibodies. In view of these observations, a preferred technique is to not let the enzymes escape and to not let immune proteins or macrophages contact the enzyme. The enzyme or coenzymes could be placed in a container such as a plastic bag or encapsulated in particles so that a substrate can diffuse in, but protein molecules cannot permeate. Peroxide could be destroyed in the bag with catalase or allowed to diffuse out to be destroyed. Also as developed above, the enzyme can be immobilized on an inert substrate such as nylon or silver. Glutaraldehyde treated tissues such as heart valves from other species have been used as substitute heart valves in human beings. Glutaraldehyde is also widely used to immobilise enzymes. Hence, glutaraldehyde can be used to immobilize and affix enzymes to surfaces for implantation where the probability of a rejection process would be very low. It is also recognised that monochromatic, dichromatic or multi-pleochromatic light can be transmitted through the earlobe and the light spectrum received on the other side to reveal the oxygen saturation of the blood. A transparent enzyme implant in the earlobe could be designed with an appropriate dye such that substrate concentration would be reflected by transmitted light.

As a result of enzyme reactions, fluorescence and phosphorescence can occur. Hence, by a suitable implant containing the enzyme and the photoactivated substance, one could detect substrate concentration by measuring the amount of light emitted to the skin by the phosphorescent reaction.

## Claims

1. A method of detecting for the presence in the body of a non-gaseous substrate characterised in that the non-gaseous substrate is volatilizable through the skin and that the method comprises introducing at the surface of the skin an enzyme selected for the substrate to be detected for a sufficient time for any of the substrate present to react with the enzyme and detecting a condition of the skin as an indication of the presence of the substrate in the body.

2. A method as claimed in Claim 1, characterised in that the enzyme is reactable with the substrate to produce a decrease in oxygen at the skin and detecting means comprising an oxygen sensitive electrode adapted for contact with the skin is used to detect the oxygen decrease at the skin as a measure of the amount of substrate.

3. A method as claimed in Claim 1, characterised in that the enzyme is reactable with the substrate to produce by-product hydrogen peroxide and detecting means comprising a polarographic anode adapted to contact the skin to detect the hydrogen peroxide at the skin as a measure of the amount of substrate or a colorimeter to detect the colour change of the skin as a measure of the amount of substrate is used to detect the condition of the skin.

4. A method as claimed in any one of Claim 1 to 3, characterised in that the substrate is an alcohol.

5. A method as claimed in Claim 4, characterised in that the substrate is ethanol.

6. A method as claimed in any preceding Claim, characterised in that the method further comprises arterializing the skin.

7. A method as claimed in Claim 6, characterised in that the skin is arterialized by chemical treatment or by heating to a temperature of 38 to 44°C.

8. A method as claimed in any preceding Claim, characterised in that the detection of the condition of the skin is by means of an electrode and the enzyme is included in the electrolyte of the electrode, or the electrode has a membrane associated with it and the enzyme is immobilized on the membrane.

9. A method as claimed in any preceding Claim, characterised in that the enzyme is selected from alcohol oxidase and alcohol dehydrogenase.

## Patentansprüche

1. Verfahren zum Feststellen eines im Körper vorhandenen nichtgasförmigen Substrats, dadurch gekennzeichnet, daß das nichtgasförmige Substrat durch die Haut verdunstbar ist und daß das Verfahren das Einführen eines Enzyms an der Hautoberfläche umfaßt, wobei das Enzym so gewählt ist, daß das Substrat über einen Zeitraum feststellbar ist, der ausreicht um alles vorhandene Substrat mit dem Enzym reagieren zu lassen, sowie das Feststellen einer Beschaffenheit der Haut als Anzeichen für das Vorhandensein des Substrats im Körper.

2. Verfahren nach Anspruch 1, dadurch gekennzeichnet, daß das Enzym mit dem Substrat auf eine Weise reagierfähig ist, daß eine Verringerung von Sauerstoff an der Haut erzeugt wird, und Mittel zum Feststellen eingesetzt werden, die eine auf Sauerstoff ansprechende, für Hautkontakt ausgelegte Elektrode enthalten und die Sauerstoffverringerung an der Haut als ein Maß für die Substratmenge feststellen.

3. Verfahren nach Anspruch 1, dadurch gekennzeichnet, daß das Enzym mit dem Substrat auf eine Weise reagierfähig ist, daß Wasserstoffperoxid als Nebenprodukt erzeugt wird, und durch eine für Hautkontakt ausgelegte polarographische Anode enthaltende Mittel zum Feststellen des Wasserstoffperoxids an dar Haut als Maß der vorhandenen Substratmenge oder ein Farbmeßgerät zum Feststellen einer farblichen Veränderung der Haut als Maß für die Substratmenge.

4. Verfahren nach einem der Ansprüche 1 bis 3, dadurch gekennzeichnet, daß das Substrat ein Alkohol ist.

5. Verfahren nach Anspruch 4, dadurch gekennzeichnet, daß das Substrat Äthanol ist.

6. Verfahren nach einem der vorhergehenden Ansprüche, dadurch gekennzeichnet, daß das Verfahren außerdem ein Umwandeln in Arterienblut für die Haut umfaßt.

7. Verfahren nach Anspruch 6, dadurch gekennzeichnet, daß die Umwandlung in Arterienblut in der Haut durch chemische Behandlung oder Erwärmung auf eine Temperatur von 38° bis 44°C erfolgt.

8. Verfahren nach einem der vorhergehenden Ansprüche, dadurch gekennzeichnet, daß das Feststellen der Hautbeschaffenheit mit Hilfe einer Elektrode geschieht, wobei das Enzym im Elektrolyt der Elektrode enthalten ist, oder mit Hilfe einer Elektrode, der eine Membran zugeordnet ist, auf der das Enzym immobilisiert angeordnet ist.

9. Verfahren nach einem der vorhergehenden Ansprüche, dadurch gekennzeichnet, daß das Enzym von Alkoholoxidase und Alkoholdehydrogenase gewählt wird.

## Revendications

1. Méthode de détection de la présence d'un substrat non gazeux dans l'organisme, caractérisée en ce que le substrat non gazeux est volatilisable à travers la peau, et en ce que la méthode consiste à introduire à la surface de la peau un enzyme choisi pour le substrat à détecter pendant une durée suffisante pour que la totalité du substrat présent réagisse avec l'enzyme, et à détecter un état de la peau comme indication de la présence du substrat dans l'organisme.

2. Méthode suivant la revendication 1, caractérisée en ce que l'enzyme est capable de réagir avec le substrat pour abaisser la quantité d'oxygène au niveau de la peau et des moyens de détection comprenant une électrode sensible à l'oxygène conçue pour le contact avec la peau sont utilisés pour détecter l'abaissement de la quantité d'oxygène au niveau de la peau comme mesure de la quantité de substrat.

3. Méthode suivant la revendication 1, caractérisée en ce que l'enzyme est apte à réagir avec le substrat en formant comme sous-produit du peroxyde d'hydrogène, et des moyens de détection comprenant une anode polarographique conçue pour entrer en contact avec la peau de manière à détecter le peroxyde d'hydrogène au niveau de la peau comme mesure de la quantité de substrat ou un colorimètre pour détecter la variation de couleur de la peau comme mesure de la quantité de substrat, sont utilisés pour détecter l'état de la peau.

4. Méthode suivant l'une quelconque des revendications 1 à 3, caractérisée en ce que le substrat est un alcool.

5. Méthode suivant la revendication 4, caractérisée en ce que le substrat est l'éthanol.

6. Méthode suivant l'une quelconque des revendications précédentes, caractérisée en ce qu'elle comporte en outre une artérialisation de la peau.

7. Méthode suivant la revendication 6, caractérisée en ce que la peau est artérialisée par traitement chimique ou par chauffage à une température de 38 à 44°C.

8. Méthode suivant l'une quelconque des revendications précédentes, caractérisée en ce que la détection de l'état de la peau est effectuée au moyen d'une électrode, et l'enzyme est inclus dans l'électrolyte de l'électrode, ou bien l'électrode est en association avec une membrane et l'enzyme est immobilisé sur la membrane.

9. Méthode suivant l'une quelconque des revendications précédentes, caractérisée en ce que l'enzyme est choisi entre une alcool-oxydase et une alcool-déshydrogénase.
